# FASCICULE DE BREVET EUROPEEN

(11) **EP 0 736 300 B1**
(45) Date de publication et mention de la délivrance du brevet: **05.03.2003**
(21) Numéro de dépôt: 96400601.9
(22) Date de dépôt: 21.03.1996
(51) Int. Cl.: A61K 31/505, A61K 47/16

(54) **Utilisation dans une composition en tant qu'activateur et/ou stabilisateur de cyclooxygénase d'au moins dérivé de pyrimidine substitué en 6**
Verwendung mindestens eines in 6-Stellung substitutierten Pirimidinderivats in einer Zusammensetzung als Cyclooxygenase-Aktivator und/oder Cyclooxygenase-Stabilisator
Use in a composition of a pyrimidine derivative substituted in position 6 as an activator and/or stabilizer of cyclooxygenase

(30) Priorité: 05.04.1995 FR 9504049
(43) Date de publication de la demande: 09.10.1996
(73) Titulaire: L'OREAL, 75008 Paris (FR)
(72) Inventeur: Michelet, Jean-François, 94000 Creteil (FR); Mahe, Yann, 91390 Morsang sur Orge (FR); Bernard, Bruno, 92200 Neuilly s/Seine (FR)
(74) Mandataire: Tezier Herman, Béatrice

(56) Documents cités:
- EP-A- 0 408 442
- EP-A- 0 459 890
- EP-A- 0 519 819
- EP-A- 0 648 488
- WO-A-95/00495
- FR-A- 2 380 775
- J LAB CLIN MED, NOV 1989, 114 (5) P575-8, UNITED STATES, XP000561593 O'BARR TP ET AL: "Effect of minoxidil on platelet function and the synthesis of prostaglandins in platelets."
- BIOCHEM PHARMACOL, MAR 1 1988, 37 (5) P867-74, ENGLAND, XP000564072 KVEDAR JC ET AL: "Selective inhibition by minoxidil of prostacyclin production by cells in culture."
- CANCER BIOCHEM BIOPHYS, OCT 1994, 14 (3) P211-20, ENGLAND, XP000561594 BILLINGS PC ET AL: "Effect of modifiers of arachidonic acid metabolism on radiation transformation and eicosanoid formation in C3H/10T1/2 cells."
- JOURNAL OF INVESTIGATIVE DERMATOLOGY, 102 (4). 1994. 631., XP000561590 GROSSMAN D ET AL: "Minoxidil affects IL-1-beta protein and mRNA levels in cultured human foreskin and psoriatic keratinocytes"

## Description

L'invention concerne l'utilisation dans une composition en tant qu'activateur et/ou stabilisateur de cyclooxygénase d'au moins un dérivé de pyrimidine substitué en 6, un kit et une composition comprenant au moins un de ces dérivés et son utilisation.

Par cyclooxygénase, il faut entendre dans la présente invention toute enzyme présentant une activité cyclooxygénase, cette enzyme pouvant présenter d'autres activités enzymatiques.
L'activité cyclooxygénase peut se définir comme l'activité enzymatique qui transforme certains acides gras polyinsaturés en produits oxygénés cyclisés qui sont en fait des endoperoxydes hautement instables qui entrent par la suite dans des voies métaboliques subséquentes.
La prostaglandine-endoperoxyde synthase (ou PGHS, EC 1.14.99.1) qui est une hémoprotéine est un exemple de ces enzymes présentant une telle activité. Elle intervient dans l'une des voies métaboliques des prostaglandines.

On sait que certains acides gras polyinsaturés, en particulier ceux présentant 20 atomes de carbone, tels que l'acide arachidonique, l'acide dihomo-γ-linolénique ou bien encore l'acide éicosapentaénoïque, peuvent être transformés *in vivo,* sous l'action de certaines enzymes spécifiques contenues dans les cellules vivantes en certains autres composés de type éicosanoïdes présents dans l'organisme.
Ainsi, il est connu que les enzymes dites cyclooxygénases génèrent, à partir des différents acides gras mentionnés ci-dessus, des éicosanoïdes de type prostaglandines, et que les enzymes dites lipoxygénases sont, quant à elles, responsables de la formation des éicosanoïdes de type leukotriènes et autres acides acycliques hydroxylés à 20 atomes de carbone.

L'implication de ces enzymes dans de nombreuses voies métaboliques et ainsi les conséquences que pourrait avoir un dérèglement de leur fonctionnement font que de nombreuses recherches ont été entreprises afin de mettre en évidence des substances ayant la capacité soit d'augmenter soit de diminuer l'activité de ces enzymes.
Dans le domaine des activateurs de cyclooxygénases, on connaît notamment les métabolites de l'acide arachidonique, le monoxyde d'azote et les composés donneurs de monoxyde d'azote, le stanozolol, les composés donneurs de glutathion, les ionophores calciques, les anthocyanosides, les bioflavonoïdes, les facteurs activateurs de plaquettes (PAF), les cytokines proinflammatoires, les endotoxines bactériennes.

De même, on peut citer la 6-chloro-2,3-dihydroxy-1,4-naphtoquinone (CNDQ), qui a la particularité d'être à la fois un inhibiteur des lipoxygénases et un stimulateur des cyclo-oxygénases (C.J. Bedord et al., The Journal of Investigative Dermatology, 81:566-571, 1983).
Cependant, la plupart de ces substances présente comme inconvénient majeur d'être à large spectre fonctionnel, ce qui entraîne qu'elles ne présentent pas en général de véritable spécificité pour la cyclo-oxygénase. A cet égard la littérature traitant de ce sujet montre une grande variété d'interprétation. Ces substances peuvent être également labiles ou leur activité dépendante de leur concentration, ce qui rend leur utilisation délicate.

La demanderesse a donc cherché de nouveaux activateurs et/ou stabilisateurs de cyclo-oxygénase.
De manière surprenante et inattendue après de longs travaux, la demanderesse a découvert que des dérivés de pyrimidine substitué en 6, et plus particulièrement les dérivés de pyrimidine 3-oxyde substitués en 6, présentent la propriété d'activer et/ou de stabiliser la cyclo-oxygénase. Cette propriété laisse suggérer que la cyclo-oxygénase est un des récepteurs de dérivés de pyrimidine substitué en 6

Cette découverte est à la base de la présente invention.

Ainsi, l'invention a pour objet l'utilisation dans une composition en tant qu'activateur et/ou stabilisateur de cyclo-oxygénase d'au moins un dérivé de pyrimidine substitué en 6.
Avantageusement, ce dérivé est un dérivé de pyrimidine 3-oxyde substitué en 6, en particulier le 2,4-diamino-6-piperidinopyrimidine 3-oxyde ou "Minoxidil".

La composition est plus particulièrement une composition cosmétique ou pharmaceutique.
Préférentiellement, la composition pharmaceutique est une composition dermatologique.

Le "Minoxidil" est connu pour ses effets anti-hypertenseur et pour sa capacité à favoriser la pousse des cheveux. Ces propriétés sont décrites dans US 4 596 812. Il est également décrit dans l'article de O'BARR et collaborateurs (J. Lab. Clin. Med., vol. 114(5), pp 575-8, 1989) comme un inhibiteur de cyclo-oxygénase et de l'agrégation plaquettaire et dans l'article de Kvedar et collaborateurs (Biochemical Pharmacology, vol.37(5), pp 867-74, 1988) comme un inhibiteur sélectif de la production de prostacycline et comme ayant un effet retardateur de la sénescencé des kératinocytes.
Par ailleurs, il est connu de nombreux autres dérivés de pyrimidine substitués en 6. Parmi ceux-ci on peut notamment citer ceux décrits dans les demandes de brevet EP 353123, EP 356271, EP 408442, EP 522964, EP 420707, EP 459890, EP 519819.

Parmi les dérivés de pyrimidine substitués en 6 utilisables selon l'invention, on peut plus particulièrement citer :
le 2,4-diamino-6-piperidinopyrimidine,
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde,
le 2-méthyl-4-amino-6-piperidinopyrimidine 3-oxyde,
le 2,4-diamino-6-butyloxypyrimidine 3-oxyde,
le 2-amino-4-propylamino-6-diméthylaminopyrimidine 3-oxyde.

Préférentiellement, on utilise selon l'invention :
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde,
le 2-méthyl-4-amino-6-piperidinopyrimidine 3-oxyde,
le 2,4-diamino-6-butyloxypyrimidine 3-oxyde,
le 2-amino-4-propylamino-6-diméthylaminopyrimidine 3-oxyde.

Et plus préférentiellement encore, on utilise selon l'invention :
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde.

Les quantités nécessaires pour activer et/ou stabiliser une cyclooxygénase sont bien entendu dépendantes de la nature du dérivé de pyrimidine substitués en 6 et de la nature de la cyclooxygénase en question. De manière générale, cette concentration est supérieure ou égale à 1nM, et de préférence supérieure à 1 µM.

Afin de déterminer l'activité des dérivés de pyrimidine substitué en 6, la demanderesse a utilisé un procédé simple et rapide de mesure consistant à incuber dans un milieu adéquat une cyclooxygénase éventuellement purifiée et un de ses substrats, en présence d'un composé à tester, et à comparer les mesures réalisées avec les résultats de mesures identiques effectuées lors de l'incubation de la cyclooxygénase, éventuellement purifiée, avec un de ses substrats en l'absence du composé à tester.

Comme indiqué précédement, l'activité enzymatique d'une cyclooxygénase se traduit par une consommation d'oxygène. Il est donc possible de suivre le déroulement de la réaction en cours par la mesure de la consommation en oxygène dans le milieu réactionnel et ainsi d'évaluer l'activité de l'enzyme par la mesure de la consommation en oxygène.
En effet, on comprend aisément que la réaction impliquant de la part de la cyclooxygénase l'utilisation de molécules d'oxygène, l'augmentation ou la diminution de cette consommation sont le reflet de la variation de l'activité de l'enzyme.
Toute méthode connue de mesure de la consommation en oxygène est bien entendu utilisable dans le procédé (voir par exemple Vanderkooi J. M. And Wilson D.F. (1986) "A new method for mesuring oxygen in biological systems" Adv. Exptl. Med. Biol. 200 : 189-193.).

La demanderesse afin de pouvoir rapidement évaluer les propriétés activatrice et/ou stabilisatrice des dérivés de pyrimidine substitué en 6 a utilisé une méthode de mesure directe et en continu de la consommation d'oxygène à l'aide d'une électrode de Clark, (CLARK, L.C., Jr. (1956). Trans. Am. Soc. Artificial Internai Organs, 2,41.), couplée à un système d'enregistrement.
Tout système d'enregistrement permettant un calcul automatique ou non de la vitesse initiale de la réaction et de la durée pendant laquelle cette vitesse initiale est maintenue peut être utilisé dans ce procédé.
La procédure générale de mesure peut ainsi être résumée :
Au temps T=0 du procédé, on introduit dans une cuve de mesure une solution tamponnée contenant une cyclooxygénase, puis on laisse l'ensemble se stabiliser pendant au moins une minute.
Au temps T=1 minute, on ajoute une quantité appropriée de substrat de l'enzyme et on enregistre la consommation en oxygène de la réaction.
Cet enregistrement permet de déduire la vitesse initiale de la réaction et la durée pendant laquelle la vitesse initiale de la réaction est maintenue.
En l'absence de toute substance autre que les ingrédients nécessaires à la réaction enzymatique, on peut ainsi déduire l'activité de base de l'enzyme. Ces données serviront de référence à l'étude des substances à tester.

Lorsque l'on teste une substance ayant potentiellement une activité sur la cyclooxygénase, la variation de la pente dans un sens positif ou négatif par rapport aux résultats obtenus avec l'enzyme seule reflète soit l'activation soit l'inhibition de l'activité de l'enzyme.
Ainsi, sachant que la cyclooxygénase a la particularité de s'autoinactiver (W. Smith and L. Marnett, Biochim. Biophy. Acta, 1083 (1991) 1-17), une variation de la durée pendant laquelle la vitesse initiale de la réaction est maintenue à son maximum traduit une stabilisation ou une déstabilisation de l'enzyme.

La cyclo-oxygénase est impliquée dans de nombreuses voies métaboliques. On peut citer entre autres, comme illustration le rôle joué par cette enzyme dans le métabolisme des prostaglandines.

On sait également qu'un même substrat, un acide gras polyinsaturé par exemple, peut être utilisé par la cyclo-oxygénase et par d'autres enzymes du métabolisme. Il se crée alors généralement des situations de compétition entre enzymes vis-à-vis du même substrat. Souvent, cela aboutit à des conséquences radicalement opposées.

Dans le cas des acides gras polyinsaturés, selon la nature de l'enzyme avec laquelle ils auront réagi en premier, on aboutit à la formation de plusieurs métabolites différents. Par exemple, avec l'acide arachidonique comme substrat, la cyclo-oxygénase et la 5-lipoxygénase conduisent respectivement à des prostaglandines et aux précurseurs des leukotriènes.

Donc, dans certains cas, à partir du même substrat, le choix de telle ou telle enzyme, ou le fait de privilégier une voie métabolique particulière, par exemple en augmentant l'activité de telle ou telle enzyme et/ou en la stabilisant, aura pour effet non seulement de privilégier une voie métabolique particulière, mais également de défavoriser la voie métabolique opposée. A ce titre on peut citer la demande EP-A-0 648 488 dans laquelle une telle démarche est utilisée pour favoriser la pousse des cheveux.
C'est ce qu'il advient lorsque l'acide arachidonique est le substrat des cyclo-oxygénases et des lipoxygénases (voir par exemple Bedord C. J. et al., J.I.D, 81 : 566-571, 1983, ou Williams K.l. dans "Nonsteroidal Anti-lnflammatory Drugs", Pharmacol. Skin. 2 : 103-117, 1989, Karger Editeur).
Ainsi, l'utilisation d'activateurs et/ou de stabilisateurs de cyclo-oxygénase peut favoriser la voie métabolique pour laquelle cette enzyme est impliquée au détriment de la voie opposée, c'est à dire celle des lipoxygénases.
Dans la mesure où, dans certaines pathologies, il est reconnu que la voie des lipoxygénases est anormalement activée, il devient intéressant de pouvoir chercher à corriger cet état de fait en cherchant à augmenter l'activité de la cyclo-oxygénase afin de diminuer par compétition l'activité de la lipoxygénase.

Ainsi, l'invention a également pour objet l'utilisation d'au moins un dérivé de pyrimidine substitué en 6, et plus particulièrement un dérivé de pyrimidine 3-oxyde substitué en 6, en tant qu'activateur et/ou stabilisateur de cyclooxygénase, dans la préparation d'une composition pharmaceutique destinée au traitement de pathologies liées soit à l'hyperactivité de la voie des lipoxygénases, soit simplement à un défaut de l'activité de la voie des cyclooxygénases ou encore pour lesquelles il s'avère utile de favoriser la voie des cyclooxygénases pour déclencher et/ou accélérer un processus.

Préférentiellement, le dérivé de pyrimidine 3-oxyde substitué en 6 utilisé dans cette préparation est le 2,4-diamino-6-piperidinopyrimidine 3-oxyde.

A cet égard on peut citer le traitement d'affections dermatologiques comme, entre autres et sans limitation, l'érythème solaire, le prurit, l'eczéma, le psoriasis, l'érythème noueux, l'urticaire, la mastocytose systémique (de Lacharrière O. et col., "Anti-inflammatoires non-stéroïdiens et peau", Thérapeutique Dermatologique, L. Dubertret éditeur, Flammarion-Médecine-Sciences Paris, 1991, pp : 698-707).

On peut également citer l'utilisation dans la préparation d'une composition pharmaceutique destinée à favoriser l'hémostase pour le traitement de plaies ou des épistaxis ou dans la préparation d'une composition anti-vieillissement.

La composition pharmaceutique peut être utilisée soit par voie locale, soit par voie systémique.

Par voie systémique, on peut citer la voie parentérale ou préférentiellement la voie entérale, plus particulièrement la voie orale.

La composition peut également être une composition cosmétique, généralement utilisée par voie locale.

Par voie locale, on préfère la voie topique, c'est à dire par application directe sur la peau, le cuir chevelu, les ongles ou les muqueuses.

Quelque soit la forme de la composition, celle-ci peut comprendre en outre tout composé habituellement utilisé en cosmétique et/ou en pharmacie.

La composition selon l'invention est une composition physiologiquement acceptable. Elle peut être anhydre ou au contraire aqueuse. Lorsqu'elle est anhydre, elle contient moins de 1% d'eau. Elle peut être constituée d'un solvant ou d'un mélange de solvants choisi plus particulièrement parmi les alcools inférieurs en C₂-C₄ comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 4 atomes de carbone. Lorsqu'elle est aqueuse, elle est constituée par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

La composition peut contenir d'autres adjuvants habituellement utilisés dans le domaine cosmétique ou pharmaceutique, tels que des agents tensioactifs, des agents émulsionnants, des agents épaississants ou gélifiants, des huiles, des agents conservateurs, des agents alcalinisants ou acidifiants bien connus dans l'état de la technique, des gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes.
Les quantités utilisées sont suffisantes pour obtenir la forme de présentation désirée, notamment de lotion plus ou moins épaissie, de gel, d'émulsion, ou de crème. La composition peut éventuellement aussi être pressurisée en aérosol ou vaporisée à partir d'un flacon pompe.

Les quantités de ces différents adjuvants sont celles classiquement utilisées dans le domaine cosmétique ou pharmaceutique, et par exemple de 0,01 % à 90 % du poids total de la composition. Ces adjuvants, selon leur nature, peuvent être introduits dans une phase grasse, dans une phase aqueuse et/ou dans des sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits végétaux, notamment ceux d'Aloe Vera.

Comme actifs lipophiles, on peut utiliser le rétinol (vitamine A) et ses dérivés, le tocophérol (vitamine E) et ses dérivés, les acides gras essentiels, les acides gras polyinsaturés, les céramides, les huiles essentielles.

La composition peut contenir en outre des enzymes, comme en particulier une cyclooxygénase. Cette cyclooxygénase est purifiée ou produite par génie génétique, d'origine animale ou végétale.

De telles compositions sont particulièrement bien adaptées au traitement des alopécies.
Le terme alopécie recouvre toute une famille d'atteintes du follicule pileux ayant pour conséquence finale la perte définitive partielle ou générale des cheveux.

La composition est plus particulièrement une composition cosmétique ou pharmaceutique.
Préférentiellement, la composition pharmaceutique est une composition dermatologique.

Les propriétés activatrice et/ou stabilisatrice des dérivés de pyrimidine substitué en 6 sont également intéressantes du fait du coût élevé de la cyclooxygénase, en particulier lorsque celle-ci est sous forme purifiée. Ainsi l'adjonction à l'enzyme d'au moins un de ces dérivés peut permettre, du fait de l'activation et/ou de la stabilisation qu'il engendre, de réduire la quantité d'enzyme utilisée.

A cet égard, l'invention concerne aussi un kit comprenant au moins une cyclooxygénase, éventuellement sous une forme purifiée, et au moins un activateur et/ou stabilisateur de celle-ci. Cet activateur et/ou stabilisateur est un dérivé de pyrimidine substitué en 6 et en particulier un dérivé de pyrimidine 3-oxyde substitué en 6. Préférentiellement ce dérivé de pyrimidine 3-oxyde substitué en 6 est du 2,4-diamino 6-piperidinopyrimidine 3-oxyde.

Les composés de ce kit peuvent être éventuellement conditionnés de manière séparée.

Ces kits sont également particulièrement bien adaptées au traitement des alopécies.

L'invention concerne plus spécifiquement un kit comprenant en outre au moins un des substrats de la cyclooxygénase.
Un tel kit peut être par exemple utilisé pour déterminer les effets inhibiteurs de cyclooxygénase d'une substance éventuellement active.

Dans certain cas, il peut s'avérer nécessaire d'augmenter l'activité des cyclooxygénases endogènes.
Ainsi, la présente invention a également pour objet une composition comprenant au moins un substrat de la cyclooxygénase et au moins un dérivé de pyrimidine substitué en 6 et plus particulièrement un dérivé de pyrimidine 3-oxyde substitué en 6. Préférentiellement, ce dérivé est le 2,4-diamino-6-piperidinopyrimidine 3-oxyde.
Dans ce cas, le dérivé utilisé peut venir activer et/ou stabiliser une cyclooxygénase présente dans tout tissu, et plus particulièrement dans la peau.

Une telle composition peut s'administrer par voie entérale, parentérale, mais de préférence par voie topique.

Préférentiellement, le substrat de la cyclooxygénase est un acide gras polyinsaturé et encore plus préférentiellement l'acide arachidonique.

Cette composition peut comprendre en outre au moins de la cyclooxygénase. Cette dernière composition trouve plusieurs applications dans des domaines très divers comme la cosmétique, la pharmacie ou encore l'agro-alimentaire. Elle s'avère très intéressante en tant que capteur d'oxygène.

En effet, lors de la réaction entre la cyclooxygénase et son substrat il y a consommation d'oxygène. Cette consommation d'oxygène peut être accrue en ajoutant au mélange au moins un activateur et/ou stabilisateur de la cyclooxygénase. On peut donc utiliser une composition de ce type afin d'éliminer l'oxygène d'un environnement afin de préserver celui-ci de l'oxydation.

L'invention a aussi comme objet l'utilisation comme capteur d'oxygène d'une composition comprenant au moins une cyclooxygénase, au moins un de ses substrats et au moins un dérivé de pyrimidine substitué en 6 et plus particulièrement un dérivé de pyrimidine 3-oxyde substitué en 6. Préférentiellement, ce dérivé est le 2,4-diamino-6-piperidinopyrimidine 3-oxyde.

Dans certaines utilisations particulières d'un tel capteur, il peut être nécessaire de prévoir d'isoler celui-ci de son environnement. C'est en particulier le cas lorsque l'on utilise le capteur ci-dessus décrit dans des secteurs comme l'agro-alimentaire ou la cosmétique. Il faut en effet avoir présent à l'esprit que la réaction enzymatique qui constitue l'élément central du capteur aboutit à la formation de composés qu'il peut être nécessaire de ne pas mélanger à l'objet à préserver de l'oxygène.

Il faut alors prévoir un conditionnement particulier permettant d'isoler physiquement le capteur d'oxygène ainsi défini du milieu à préserver de l'oxygène. Dans un mode particulier de l'invention, le capteur d'oxygène est isolé du produit contenu dans le conditionnement par une membrane perméable au gaz et imperméable aux liquides notamment ceux entrant dans la composition de l'émulsion selon l'invention.

La membrane perméable au gaz peut être, par exemple, celle définie dans le document FR-A-2 671 055.

Le capteur d'oxygène isolé par la membrane peut être, dans le dispositif de conditionnement, solidaire des parois ou non solidaire de ces parois et noyé dans la composition.

Quelque soit la composition envisagée selon l'invention, l'homme du métier sait en adapter la concentration des divers composants, sachant que l'activité basale de l'enzyme peut varier de manière aléatoire selon le lot utilisé et que cette activité conditionne les quantités utilisées de substrat et/ou d'activateur et/ou de stabilisateur.

On va maintenant donner à titre d'illustration des exemples de mesures de l'activité cyclooxygénase en présence ou en l'absence de dérivés et en particulier de dérivés de pyrimidine, qui ne sauraient limiter en aucune façon la portée de l'invention.

### Exemple 1 :

Mesure du pouvoir activateur et/ou stabilisateur de dérivés de pyrimidine substitués en 6, à une concentration donnée.

### Principes généraux de mesure :

On mesure la quantité d'oxygène nécessaire à l'oxydation de l'acide arachidonique par l'activité cyclooxygénase de la prostaglandine-endoperoxyde synthase, en présence ou en absence du dérivé à tester.

Les mesures de la consommation d'oxygène sont effectuées avec une électrode de Clark raccordée à un oxymétre YSI 5300 de marque Yellow Spring Instruments.
Ces mesures sont effectuées en chambre ouverte sous agitation constante à la température de 37°C.
Si l'on utilise un enregistreur graphique, la mesure de la consommation en oxygène se matérialise sous la forme d'une courbe, dont la pente maximale permet de déduire la vitesse initiale de la réaction, et à partir de laquelle il est possible de calculer la durée pendant laquelle la vitesse initiale de la réaction est maintenue.

La courbe ainsi obtenue, en l'absence de toute substance autre que les ingrédients nécessaires à la réaction enzymatique, donne l'activité de base de l'enzyme. On peut déterminer dans ces conditions la vitesse initiale et la durée pendant laquelle cette vitesse est maintenue, dans une réaction ne comportant que l'enzyme et son substrat.
Ces données serviront de référence à l'étude des dérivés à tester.

La mesure de l'activité des dérivés à tester s'effectue dans les mêmes conditions, en ajoutant au milieu réactionnel le dérivé à tester. Ce sont la variation de la pente et la variation de la durée pendant laquelle la vitesse maximale est maintenue qui permettent d'évaluer l'activité du dérivé à tester vis à vis de la cyclooxygénase.

### Préparation aux mesures :

On prépare une solution de TRIS 0,1 M et d'EDTA 5 mM à pH = 8,00 (solution TE).
Les mesures s'effectuent dans une solution tamponnée (Tampon TEA) composée de 9 volumes de solution TE et 1 volume d'alcool à 20%.

Le substrat est préparé sous la forme d'une solution mère d'arachidonate de potassium selon le protocole du fournisseur (lnterchim, France).
La solution ainsi obtenue titre à 46 mM en acide arachidonique. Elle peut être stockée à 4°C pour 24 heures.

L'enzyme utilisée est de la prostaglandine-endoperoxyde synthase (PGHS), isolée de glandes séminales de mouton, vendue par la société Cayman Chemical sous la référence 60100.

Les dérivés à tester sont préparés sous forme de solution mère titrant à 5 mM dans un mélange eau/alcool (80/20) et testés avec un même lot d'enzyme.

### Mesures :

### Activités de base de l'enzyme :

A t = 0, on introduit dans la chambre de mesure 380 µl de tampon TEA préchauffé à 37°C, qu'on laisse s'équilibrer au moins une minute.
A t = 1, on introduit 300 unités d'enzyme (PGHS).
L'enregistrement est déclenché, et on laisse l'ensemble se stabiliser de nouveau pendant une minute. L'enregistrement obtenu donne le niveau de base de la réaction.

Au bout d'une minute supplémentaire, on introduit 10 µl de substrat et l'on enregistre la consommation en oxygène pendant 2 à 3 minutes.

On détermine ainsi la vitesse initiale de la réaction et la durée pendant laquelle cette vitesse est maintenue. Ces données serviront de référence aux mesures de l'activité des dérivés à tester.

### Activités des dérivés à tester :

Les conditions d'expérimentation sont identiques aux précédentes, si ce n'est que le tampon TEA préchauffé à 37°C est remplacé par un tampon identique contenant le dérivé à tester à la concentration de 0,5 mM.

### Résultats :

Ces résultats sont exprimés en % par rapport aux valeurs obtenues avec le témoin.

| Dérivés | Activation | Stabilisation |
|---|---|---|
| Témoin | +0% | +0% |
| 2,4-diamino-6-piperidinopyrimidine 3-oxyde | +58% | +14% |
| 2,4-diamino-6-piperidinopyrimidine | -14% | +35% |
| 2,4-dipropylamino-6-diméthylaminopyrimidine 3-oxyde. | +171% | +28% |
| 2-amino-4-propylamino-6-piperidinopyrimidine 3-oxyde | +173% | +17% |
| 2-méthyl-4-amino-6-piperidinopyrimidine 3-oxyde | + 24% | 0% |
| 2,4-diamino-6-butyloxypyrimidine 3-oxyde | +13% | 0% |
| 2-amino-4-propylamino-6-diméthylaminopyrimidine | +117% | 0% |
| 3-oxyde | | |
| Indométhacine¹ | -100% | -100% |

| | | |
|---|---|---|
| ¹ : le 1-(4-chlorobenzoyl)-5-methoxy-2 methyl-1H-indole 3 acide acétique ou indométhacine est un inhibiteur connu de l'activité cyclooxygénase. (H.P Rang / M.M.Dale Pharmacology second édition 1991. Churchill Livingstone édition). Dans les conditions de l'expérience, on retrouve bien cet effet inhibiteur. | | |

Ces résultats montrent que des dérivés de pyrimidine substitués en 6 présentent des propriétés d'activateur et/ou de stabilisateur de la cyclooxygénase.

### Exemple 2 :

Calcul de la concentration activatrice à 50% (AC 50), du 2,4-diamino-6-piperidinopyrimidine 3-oxyde pour l'activation de la Prostaglandine endoperoxyde synthase.

| 2,4-diamino-6-piperidinopyrimidine 3-oxyde (µM) | Activation (% du témoin) |
|---|---|
| 0 | 0 |
| 15,6 | 23 |
| 62,5 | 61 |
| 125 | 90 |
| 250 | 102 |
| 500 | 146 |
| 1000 | 181 |

Par calcul statistique (régression log-linéaire, le coefficient de corrélation étant de 0,98 et la significativité de liaison inférieure ou égale à 0,01), on trouve une AC 50 pour 40 µM pour le 2,4-diamino-6-piperidinopyrimidine 3-oxyde.

### Exemple 3 : Crème dermique

On prépare une crème dermique par mélange des ingrédients suivants :
- 2,4-diamino-6-piperidinopyrimidine 3-oxyde 0,5 g
- Ceteareth 30 7 g
- Stéarate de glycéryle 2 g
- Alcool cétylique 1,5 g
- Polydiméthylsiloxane 1,5 g
- Huile de paraffine 15 g
- Glycérine codex pure 20 g
- Conservateurs q.s.
- Eau déminéralisée q.s.p. 100 g

### Exemple 4 : Lotion dermique à pulvériser

On prépare une lotion dermique à pulvériser par mélange des ingrédients suivants :
- 2,4-dipropylamino-6-diméthylaminopyrimidine 3-oxyde 0,025 g
- Ethanol 30 g
- Eau déminéralisée q.s.p. 100 g

### Exemple 5 : Solution injectable

On prépare une solution injectable par voie intradermique par mélange des ingrédients suivants :
- 2-amino-4-propylamino-6-piperidinopyrimidine 3-oxyde 0,01 mg
- Sérum physiologique (NaCI 9 g/H2O qsp 100 ml) q.s.p. 1 ml

## Revendications

1. Utilisation non thérapeutique dans une composition, comme activateur et/ou stabilisateur de cyclo-oxygénase, d'au moins un dérivé de pyrimidine substitué en 6.

2. Utilisation selon la revendication 1, **caractérisée par le fait que** le dérivé de pyrimidine substitué en 6 est un dérivé de pyrimidine 3-oxyde substitué en 6.

3. Utilisation selon l'une quelconque des revendications 1 ou 2, **caractérisée par le fait que** le dérivé de pyrimidine substitué en 6 est choisi parmi :
le 2,4-diamino-6-piperidinopyrimidine,
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde,
le 2-méthyl-4-amino-6-piperidinopyrimidine 3-oxyde,
le 2,4-diamino-6-butyloxypyrimidine 3-oxyde,
le 2-amino-4-propylamino-6-diméthylaminopyrimidine 3-oxyde.

4. Utilisation selon la revendication 3, **caractérisée par le fait que** le dérivé de pyrimidine substitué en 6 est choisi parmi :
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde.

5. Utilisation selon l'une quelconque des revendications précédentes, **caractérisée par le fait que** la composition est une composition cosmétique.

6. Composition, **caractérisée par le fait qu'**elle comprend au moins un des substrats de la cyclo-oxygénase, au moins un dérivé de pyrimidine substitué en 6 et au moins une cyclo-oxygénase.

7. Composition selon la revendication précédente, **caractérisée par le fait que** dérivé de pyrimidine substitué en 6 est un dérivé de pyrimidine 3-oxyde substitué en 6.

8. Composition selon la revendication 6 ou 7, **caractérisée par le fait que** le dérivé de pyrimidine substitué en 6 est choisi parmi :
le 2,4-diamino-6-piperidinopyrimidine,
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde,
le 2-méthyl-4-amino-6-piperidinopyrimidine 3-oxyde,
le 2,4-diamino-6-butyloxypyrimidine 3-oxyde,
le 2-amino-4-propylamino-6-diméthylaminopyrimidine 3-oxyde.

9. Composition selon la revendication 8, **caractérisée par le fait que** le dérivé de pyrimidine substitué en 6 est choisi parmi :
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde.

10. Composition selon l'une quelconque des revendications 6 à 9, **caractérisée par le fait que** la cyclo-oxygénase est sous une forme purifiée.

11. Utilisation d'au moins un dérivé de pyrimidine substitué en 6 dans la préparation d'une composition pharmaceutique, destinée au traitement de pathologies liées soit à l'hyperactivité de la voie des lipoxygénases, soit à un défaut de l'activité de la voie des cyclo-oxygénases, soit pour lesquelles il s'avère utile de favoriser la voie des cyclo-oxygénases pour déclencher ou accélérer un processus, à l'exception d'une utilisation antivieillissement et d'une utilisation pour favoriser la pousse des cheveux.

12. Utilisation d'au moins un dérivé de pyrimidine substitué en 6 dans la préparation d'un médicament destiné au traitement de l'érythème solaire, du prurit, de l'eczéma, du psoriasis, de l'érythème noueux, de l'urticaire, de la mastocytose systémique, destiné à favoriser l'hémostase, la cicatrisation.

13. Utilisation selon l'une quelconque des revendications 11 ou 12, **caractérisée par le fait que** le dérivé de pyrimidine substitué en 6 est un dérivé de pyrimidine 3-oxyde substitué en 6.

14. Utilisation selon l'une quelconque des revendications 11 à 13, **caractérisée par le fait que** le dérivé de pyrimidine substitué en 6 est choisi parmi :
le 2,4-diamino-6-piperidinopyrimidine,
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde,
le 2-méthyl-4-amino-6-piperidinopyrimidine 3-oxyde,
le 2,4-diamino-6-butyloxypyrimidine 3-oxyde,
le 2-amino-4-propylamino-6-diméthylaminopyrimidine 3-oxyde.

15. Utilisation selon la revendication 14, **caractérisée par le fait que** le dérivé de pyrimidine substitué en 6 est choisi parmi :
le 2,4-diamino-6-piperidinopyrimidine,
le 2,4-dipropylamino-6-diméthylaminopyrimidine 3-oxyde,
le 2-amino-4-propylamino-6-piperidinopyrimidine 3-oxyde.

16. Utilisation du 2,4-diamino-6-piperidinopyrimidine, du 2,4-diamino-6-piperidinopyrimidine 3-oxyde et du 2,4-diamino-6-butyloxypyrimidine 3-oxyde, dans la préparation d'une composition pharmaceutique destinée au traitement de pathologies liées soit à l'hyperactivité de la voie des lipoxygénases, soit à un défaut de l'activité de la voie des cyclo-oxygénases, soit pour lesquelles il s'avère utile de favoriser la voie des cyclo-oxygénases pour déclencher ou accélérer un processus.

17. Utilisation d'au moins un dérivé de pyrimidine substitué en 6, à l'exception du 2,4-diamino-6-piperidinopyrimidine 3-oxyde, dans la préparation d'un médicament destiné au traitement de l'érythème solaire, du prurit, de l'eczéma, du psoriasis, de l'érythème noueux, de l'urticaire, de la mastocytose systémique, destiné à favoriser l'hémostase, la cicatrisation ou dans la préparation d'une composition antivieillissement.

18. Utilisation selon l'une quelconque des revendications 16 ou 17, **caractérisée par le fait que** le dérivé de pyrimidine substitué en 6 est un dérivé de pyrimidine 3-oxyde substitué en 6.

19. Utilisation selon l'une quelconque des revendications 16 à 18, **caractérisée par le fait que** le dérivé de pyrimidine substitué en 6 est choisi parmi :
le 2,4-dipropylamino-6-diméthylaminopyrimidine 3-oxyde,
le 2-amino-4-propylamino-6-piperidinopyrimidine 3-oxyde,
le 2-méthyl-4-amino-6-piperidinopyrimidine 3-oxyde,
le 2,4-diamino-6-butyloxypyrimidine 3-oxyde,
le 2-amino-4-propylamino-6-diméthylaminopyrimidine 3-oxyde.

20. Utilisation selon la revendication 19, **caractérisée par le fait que** le dérivé de pyrimidine substitué en 6 est choisi parmi :
le 2,4-dipropylamino-6-diméthylaminopyrimidine 3-oxyde,
le 2-amino-4-propylamino-6-piperidinopyrimidine 3-oxyde.

21. Composition ou kit, **caractérisé par le fait qu'**il comprend au moins une cyclo-oxygénase et au moins un dérivé de pyrimidine substitué en 6.

22. Composition ou kit selon la revendication précédente, **caractérisé par le fait que** le dérivé de pyrimidine substitué en 6 est un dérivé de pyrimidine 3-oxyde substitué en 6.

23. Composition ou kit selon l'une des revendications 21 et 22, **caractérisé par le fait que** le dérivé de pyrimidine substitué en 6 est choisi parmi :
le 2,4-diamino-6-piperidinopyrimidine,
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde,
le 2-méthyl-4-amino-6-piperidinopyrimidine 3-oxyde,
le 2,4-diamino-6-butyloxypyrimidine 3-oxyde,
le 2-amino-4-propylamino-6-diméthylaminopyrimidine 3-oxyde.

24. Composition ou kit selon la revendication précédente, **caractérisé par le fait que** le dérivé de pyrimidine substitué en 6 est choisi parmi :
le 2,4-diamino-6-piperidinopyrimidine 3-oxyde,
le 2,4-dipropylamino-6-diméthylaminopyrimidine 3-oxyde,
le 2-amino-4-propylamino-6-piperidinopyrimidine 3-oxyde.

25. Composition ou kit selon l'une quelconque des revendications 21 à 24, **caractérisé par le fait que** la cyclo-oxygénase est sous une forme purifiée.

26. Composition ou kit selon l'une quelconque des revendications 21 à 25, **caractérisé par le fait qu'**il contient au moins un substrat de la cyclo-oxygénase.

27. Utilisation d'une composition ou d'un kit selon la revendication 26 comme capteur d'oxygène.

28. Composition selon l'une quelconque des revendications 21 à 26, destinée au traitement de l'alopécie.

## Patentansprüche

1. Nicht therapeutische Verwendung mindestens eines in 6-Stellung substituierten Pyrimidinderivats in einer Zusammensetzung als Cyclooxygenase-Aktivator und/oder Cyclooxygenase-Stabilisator.

2. Verwendung nach Anspruch 1, **dadurch gekennzeichnet, dass** das in 6-Stellung substituierte Pyrimidinderivat ein in 6-Stellung substituiertes Pyrimidin-3-oxid-Derivat ist.

3. Verwendung nach einem der Ansprüche 1 oder 2, **dadurch gekennzeichnet, dass** das in 6-Stellung substituierte Pyrimidinderivat ausgewählt ist unter:
2,4-Diamino-6-piperidino-pyrimidin,
2,4-Diamino-6-piperidino-pyrimidin-3-oxid,
2,4-Dipropylamino-6-dimethylamino-pyrimidin-3-oxid,
2-Amino-4-propylamino-6-piperidino-pyrimidin-3-oxid,
2-Methyl-4-amino-6-piperidino-pyrimidin-3-oxid,
2,4-Diamino-6-butyloxy-pyrimidin-3-oxid und
2-Amino-4-propylamino-6-dimethylamino-pyrimidin-3-oxid.

4. Verwendung nach Anspruch 3, **dadurch gekennzeichnet, dass** das in 6-Stellung substituierte Pyrimidinderivat ausgewählt ist unter:
2,4-Diamino-6-piperidino-pyrimidin-3-oxid,
2,4-Dipropylamino-6-dimethylamino-pyrimidin-3-oxid und
2-Amino-4-propylamino-6-piperidino-pyrimidin-3-oxid.

5. Verwendung nach einem der vorhergehenden Ansprüche, **dadurch gekennzeichnet, dass** die Zusammensetzung eine kosmetische Zusammensetzung ist.

6. Zusammensetzung, **dadurch gekennzeichnet, dass** sie mindestens eines der Substrate der Cyclooxygenase, mindestens ein in 6-Stellung substituiertes Pyrimidinderivat und mindestens eine Cyclooxygenase enthält.

7. Zusammensetzung nach dem vorhergehenden Anspruch, **dadurch gekennzeichnet, dass** das in 6-Stellung substituierte Pyrimidinderivat ein in 6-Stellung substituiertes Pyrimidin-3-oxid-Derivat ist.

8. Zusammensetzung nach Anspruch 6 oder 7, **dadurch gekennzeichnet, dass** das in 6-Stellung substituierte Pyrimidinderivat ausgewählt ist unter:
2,4-Diamino-6-piperidino-pyrimidin,
2,4-Diamino-6-piperidino-pyrimidin-3-oxid,
2,4-Dipropylamino-6-dimethylamino-pyrimidin-3-oxid,
2-Amino-4-propylamino-6-piperidino-pyrimidin-3-oxid,
2-Methyl-4-amino-6-piperidino-pyrimidin-3-Oxid,
2,4-Diamino-6-butyloxy-pyrimidin-3-oxid und
2-Amino-4-propylamino-6-dimethylamino-pyrimidin-3-oxid.

9. Zusammmensetzung nach Anspruch 8, **dadurch gekennzeichnet, dass** das in 6-Stellung substituierte Pyrimidinderivat ausgewählt ist unter:
2,4-Diamino-6-piperidino-pyrimidin-3-oxid,
2,4-Dipropylamino-6-dimethylamino-pyrimidin-3-oxid und
2-Amino-4-propylamino-6-piperidino-pyrimidin-3-oxid.

10. Zusammensetzung nach einem der Ansprüche 6 bis 9, **dadurch gekennzeichnet, dass** die Cyclooxygenase in einer gereinigten Form vorliegt.

11. Verwendung mindestens eines in 6-Stellung substituierten Pyrimidinderivats bei der Herstellung einer pharmazeutischen Zusammensetzung, die zur Behandlung von Erkrankungen bestimmt ist, die entweder mit der Hyperaktivität des Weges der Lipoxygenasen oder mit einer mangelnden Aktivität des Weges der Cyclooxygenasen verbunden sind, oder bei denen es sich als nützlich erweist, den Weg der Cyclooxygenasen zu begünstigen, um einen Prozess auszulösen oder zu beschleunigen, mit Ausnahme einer Verwendung gegen Alterung oder einer Verwendung zur Förderung des Haarwuchses.

12. Verwendung mindestens eines in 6-Stellung substituierten Pyrimidinderivats bei der Herstellung eines Arzneimittels, das zur Behandlung von Sonnenbrand, Juckreiz, Ekzemen, Psoriasis, Erythema nodosum, Urtikaria und systemischer Mastozytose bestimmt ist oder das die Hämostase und die Wundheilung begünstigen soll.

13. Verwendung nach einem der Ansprüche 11 oder 12, **dadurch gekennzeichnet, dass** das in 6-Stellung substituierte Pyrimidinderivat ein in 6-Stellung substituiertes Pyrimidin-3-oxid-derivat ist.

14. Verwendung nach einem der Ansprüche 11 bis 13, **dadurch gekennzeichnet, dass** das in 6-Stellung substituierte Pyrimidinderivat ausgewählt ist unter:
2,4-Diamino-6-piperidino-pyrimidin,
2,4-Diamino-6-piperidino-pyrimidin-3-oxid,
2,4-Dipropylamino-6-dimethylamino-pyrimidin-3-oxid,
2-Amino-4-propylamino-6-piperidino-pyrimidin-3-oxid,
2-Methyl-4-amino-6-piperidino-pyrimidin-3 -oxid,
2,4-Diamino-6-butyloxy-pyrimidin-3-Oxid und
2-Amino-4-propylamino-6-dimethylamino-pyrimidin-3-oxid.

15. Verwendung nach Anspruch 14, **dadurch gekennzeichnet, dass** das in 6-Stellung substituierte Pyrimidinderivat ausgewählt ist unter:
2,4-Diamino-6-piperidino-pyrimidin,
2,4-Dipropylamino-6-dimethylamino-pyrimidin-3-oxid und
2-Amino-4-propylamino-6-piperidino-pyrimidin-3-oxid.

16. Verwendung von 2,4-Diamino-6-piperidino-pyrimdin, 2,4-Diamino-6-piperidino-pyrimdin-3-oxid und 2,4-Diamino-6-butyloxy-pyrimidin-3-oxid bei der Herstellung einer pharmazeutischen Zusammensetzung, die zu Behandlung von Krankheiten bestimmt ist, die entweder mit der Hyperaktivität des Weges der Lipoxygenasen oder mit einer mangelnden Aktivität des Weges der Cyclooxygenasen verbunden sind oder bei denen es sich als nützlich erweist, den Weg der Cyclooxygenasen zu begünstigen, um einen Prozess auszulösen oder zu beschleunigen.

17. Verwendung mindestens eines in 6-Stellung substituierten Pyrimidinderivats mit Ausnahme von 2,4-Diamino-6-piperidino-pyrimdin-3-oxid bei der Herstellung eines Arzneimittels, das zur Behandlung von Sonnenbrand, Juckreiz, Ekzemen, Psoriasis, Erythema nodosum, Urtikaria und systemischer Mastozytose bestimmt ist oder das die Hämostase und die Wundheilung begünstigen soll, oder bei der Herstellung einer Zusammensetzung gegen Alterung.

18. Verwendung nach einem der Ansprüche 16 oder 17, **dadurch gekennzeichnet, dass** das in 6-Stellung substituierte Pyrimidinderivat ein in 6-Stellung substituiertes Pyrimidin-3-oxid-Derivat ist.

19. Verwendung nach einem der Ansprüche 16 bis 18, **dadurch gekennzeichnet, dass** das in 6-Stellung substituierte Pyrimidinderivat ausgewählt ist unter:
2,4-Dipropylamino-6-dimethylamino-pyrimidin-3-oxid,
2-Amino-4-propylamino-6-piperidino-pyrimidin-3-Oxid,
2-Methyl-4-amino-6-piperidino-pyrimidin-3-oxid,
2,4-Diamino-6-butyloxy-pyrimidin-3-oxid und
2-Amino-4-propylamino-6-dimethylamino-pyrimidin-3-Oxid.

20. Verwendung nach Anspruch 19, **dadurch gekennzeichnet, dass** das in 6-Stellung substituierte Pyrimidinderivat ausgewählt ist unter:
2,4-Dipropylamino-6-dimethylamino-pyrimidin-3-oxid und
2-Amino-4-propylamino-6-piperidino-pyrimidin-3-oxid.

21. Zusammensetzung oder Kit, **dadurch gekennzeichnet, dass** sie bzw. er mindestens eine Cyclooxygenase und mindestens ein in 6-Stellung substituiertes Pyrimidinderivat enthält.

22. Zusammensetzung oder Kit nach dem vorhergehenden Anspruch, **dadurch gekennzeichnet, dass** das in 6-Stellung substituierte Pyrimidinderivat ein in 6-Stellung substituiertes Pyrimidin-3-oxid-Derivat ist.

23. Zusammensetzung oder Kit nach einem der Ansprüche 21 und 22, **dadurch gekennzeichnet, dass** das in 6-Stellung substituierte Pyrimidinderivat ausgewählt ist unter:
2 ,4-Diamino-6-piperidino-pyrimidin,
2,4-Diamino-6-piperidino-pyrimidin-3-oxid,
2,4-Dipropylamino-6-dimethylamino-pyrimidin-3-oxid,
2-Amino-4-propylamino-6-piperidino-pyrimidin-3-oxid,
2-Methyl-4-amino-6-piperidino-pyrimidin-3-oxid,
2,4-Diamino-6-butyloxy-pyrimidin-3-oxid und
2-Amino-4-propylamino-6-dimethylamino-pyrimidin-3-oxid.

24. Zusammensetzung oder Kit nach dem vorhergehenden Anspruch, **dadurch gekennzeichnet, dass** das in 6-Stellung substituierte Pyrimidinderivat ausgewählt ist unter:
2,4-Diamino-6-piperidino-pyrimidin-3-oxid,
2,4-Dipropylamino-6-dimethylamino-pyrimidin-3-oxid und
2-Amino-4-propylamino-6-piperidino-pyrimidin-3-oxid.

25. Zusammensetzung oder Kit nach einem der Ansprüche 21 bis 24, **dadurch gekennzeichnet, dass** die Cyclooxygenase in einer gereinigten Form vorliegt.

26. Zusammensetzung oder Kit nach einem der Ansprüche 21 bis 25, **dadurch gekennzeichnet, dass** sie bzw. er mindestens ein Substrat der Cyclooxygenase enthält.

27. Verwendung einer Zusammensetzung oder eines Kits nach Anspruch 26 als Sauerstofffänger.

28. Zusammensetzung nach einem der Ansprüche 21 bis 26, die zur Behandlung von Alopezie bestimmt ist.

## Claims

1. Non-therapeutic use, in a composition, as cyclooxygenase activator and/or stabilizer, of at least one pyrimidine derivative substituted at the 6-position.

2. Use according to Claim 1, **characterized in that** the pyrimidine derivative substituted at the 6-position is a pyrimidine 3-oxide derivative substituted at the 6-position.

3. Use according to either of Claims 1 and 2, **characterized in that** the pyrimidine derivative substituted at the 6-position is chosen from:
2,4-diamino-6-piperidinopyrimidine,
2,4-diamino-6-piperidinopyrimidine 3-oxide,
2,4-dipropylamino-6-dimethylaminopyrimidine 3-oxide,
2-amino-4-propylamino-6-piperidinopyrimidine 3-oxide,
2-methyl-4-amino-6-piperidinopyrimidine 3-oxide,
2,4-diamino-6-butyloxypyrimidine 3-oxide,
2-amino-4-propylamino-6-dimethylaminopyrimidine 3-oxide.

4. Use according to Claim 3, **characterized in that** the pyrimidine derivative substituted at the 6-position is chosen from:
2,4-diamino-6-piperidinopyrimidine 3-oxide,
2,4-dipropylamino-6-dimethylaminopyrimidine 3-oxide,
2-amino-4-propylamino-6-piperidinopyrimidine 3-oxide.

5. Use according to any one of the preceding claims, **characterized in that** the composition is a cosmetic composition.

6. Composition, **characterized in that** it comprises at least one of the cyclooxygenase substrates, and at least one pyrimidine derivative substituted at the 6-position and at least one cyclooxygenase.

7. Composition according to the preceding claim, **characterized in that** the pyrimidine derivative substituted at the 6-position is a pyrimidine 3-oxide derivative substituted at the 6-position.

8. Composition according to Claim 6 or 7, **characterized in that** the pyrimidine derivative substituted at the 6-position is chosen from:
2,4-diamino-6-piperidinopyrimidine,
2,4-diamino-6-piperidinopyrimidine 3-oxide,
2,4-dipropylamino-6-dimethylaminopyrimidine 3-oxide,
2-amino-4-propylamino-6-piperidinopyrimidine 3-oxide,
2-methyl-4-amino-6-piperidinopyrimidine 3-oxide,
2,4-diamino-6-butyloxypyrimidine 3-oxide, .
2-amino-4-propylamino-6-dimethylaminopyrimidine 3-oxide.

9. Composition according to Claim 8, **characterized in that** the pyrimidine derivative substituted at the 6-position is chosen from:
2,4-diamino-6-piperidinopyrimidine 3-oxide,
2,4-dipropylamino-6-dimethylaminopyrimidine 3-oxide,
2-amino-4-propylamino-6-piperidinopyrimidine 3-oxide.

10. Composition according to any one of Claims 6 to 9, **characterized in that** the cyclooxygenase is in a purified form.

11. Use of at least one pyrimidine derivative substituted at the 6-position, in the preparation of a pharmaceutical composition intended for the treatment of pathologies linked either to hyperactivity of the lipoxygenase pathway or to a deficiency in the activity of the cyclooxygenase pathway, or alternatively for which it is found to be useful to favour the cyclooxygenase pathway in order to initiate or accelerate a process with the exception of an anti-ageing use and a use for promoting hair growth.

12. Use of at least one pyrimidine derivative substituted at the 6-position, in the preparation of a medicinal product intended for the treatment of solar erythema, pruritus, eczema, psoriasis, erythema nodosum, urticaria and systemic mastocytosis, intended to promote haemostasis and cicatrization.

13. Use according to either of Claims 11 and 12, **characterized in that** the pyrimidine derivative substituted at the 6-position is a pyrimidine 3-oxide derivative substituted at the 6-position.

14. Use according to any one of Claims 11 to 13, **characterized in that** the pyrimidine derivative substituted at the 6-position is chosen from:
2,4-diamino-6-piperidinopyrimidine,
2,4-diamino-6-piperidinopyrimidine 3-oxide,
2,4-dipropylamino-6-dimethylaminopyrimidine 3-oxide,
2-amino-4-propylamino-6-piperidinopyrimidine 3-oxide,
2-methyl-4-amino-6-piperidinopyrimidine 3-oxide,
2,4-diamino-6-butyloxypyrimidine 3-oxide,
2-amino-4-propylamino-6-dimethylaminopyrimidine 3-oxide.

15. Use according to Claim 14, **characterized in that** the pyrimidine derivative substituted at the 6-position is chosen from:
2,4-diamino-6-piperidinopyrimidine,
2,4-dipropylamino-6-dimethylaminopyrimidine 3-oxide,
2-amino-4-propylamino-6-piperidinopyrimidine 3-oxide.

16. Use of 2,4-diamino-6-piperidinopyrimidine, 2,4-diamino-6-piperidinopyrimidine 3-oxide and 2,4-diamino-6-butyloxypyrimidine 3-oxide, in the preparation of a pharmaceutical composition intended for the treatment of pathologies linked either to hyperactivity of the lipoxygenase pathway or to a deficiency in the activity of the cyclooxygenase pathway, or alternatively for which it is found to be useful to favour the cyclooxygenase pathway in order to initiate or accelerate a process.

17. Use of at least one pyrimidine derivative substituted at the 6-position, with the exception of 2,4-diamino-6-piperidinopyrimidine 3-oxide, in the preparation of a medicinal product intended for the treatment of solar erythema, pruritus, eczema, psoriasis, erythema nodosum, urticaria and systemic mastocytosis, intended to promote haemostasis and cicatrization, or in the preparation of an anti-ageing composition.

18. Use according to either of Claims 16 and 17, **characterized in that** the pyrimidine derivative substituted at the 6-position is a pyrimidine 3-oxide derivative substituted at the 6-position.

19. Use according to any one of Claims 16 to 18, **characterized in that** the pyrimidine derivative substituted at the 6-position is chosen from:
2,4-dipropylamino-6-dimethylaminopyrimidine 3-oxide,
2-amino-4-propylamino-6-piperidinopyrimidine 3-oxide,
2-methyl-4-amino-6-piperidinopyrimidine 3-oxide,
2,4-diamino-6-butyloxypyrimidine 3-oxide,
2-amino-4-propylamino-6=dimethylaminopyrimidine 3-oxide.

20. Use according to Claim 19, **characterized in that** the pyrimidine derivative substituted at the 6-position is chosen from:
2,4-dipropylamino-6-dimethylaminopyrimidine 3-oxide,
2-amino-4-propylamino-6-piperidinopyrimidine 3-oxide.

21. Composition or kit, **characterized in that** it comprises at least one cyclooxygenase and at least one pyrimidine derivative substituted at the 6-position.

22. Composition or kit according to the preceding claim, **characterized in that** the pyrimidine derivative substituted at the 6-position is a pyrimidine 3-oxide derivative substituted at the 6-position.

23. Composition or kit according to either of Claims 21 and 22, **characterized in that** the pyrimidine derivative substituted at the 6-position is chosen from:
2,4-diamino-6-piperidinopyrimidine,
2,4-diamino-6-piperidinopyrimidine 3-oxide,
2,4-dipropylamino-6-dimethylaminopyrimidine 3-oxide,
2-amino-4-propylamino-6-piperidinopyrimidine 3-oxide,
2-methyl-4-amino-6-piperidinopyrimidine 3-oxide,
2,4-diamino-6-butyloxypyrimidine 3-oxide,
2-amino-4-propylamino-6-dimethylaminopyrimidine 3-oxide.

24. Composition or kit according to the preceding claim, **characterized in that** the pyrimidine derivative substituted at the 6-position is chosen from:
2,4-diamino-6-piperidinopyrimidine 3-oxide,
2,4-dipropylamino-6-dimethylaminopyrimidine 3-oxide,
2-amino-4-propylamino-6-piperidinopyrimidine 3-oxide.

25. Composition or kit according to any one of Claims 21 to 24, **characterized in that** the cyclooxygenase is in a purified form.

26. Composition or kit according to any one of Claims 21 to 25, **characterized in that** it contains at least one cyclooxygenase substrate.

27. Use of a composition or of a kit according to Claim 26, as an oxygen scavenger.

28. Composition according to any one of Claims 21 to 26, intended for the treatment of alopecia.
